Europäisches Patentamt

European Patent Office

Office européen des brevets

(19)

(11) **EP 0 764 223 B1**

(12) **EUROPÄISCHE PATENTSCHRIFT**

(45) Veröffentlichungstag und Bekanntmachung des Hinweises auf die Patenterteilung:
**19.08.1998 Patentblatt 1998/34**

(21) Anmeldenummer: **95923226.5**

(22) Anmeldetag: **03.06.1995**

(51) Int Cl.6: **D04H 1/64**, D06M 14/26, A61L 15/00

(86) Internationale Anmeldenummer:
**PCT/EP95/02114**

(87) Internationale Veröffentlichungsnummer:
**WO 95/33878 (14.12.1995 Gazette 1995/53)**

(54) **VERFAHREN ZUR HERSTELLUNG EINES WASSERABSORBIERENDEN FLÄCHENGEBILDES UND VERWENDUNG**

METHOD OF PRODUCING A WATER-ABSORBING FLAT ARTICLE AND USE THEREOF

PROCEDE DE FABRICATION D'UN PRODUIT PLAT HYDROABSORBANT ET SON UTILISATION

(84) Benannte Vertragsstaaten:
**DE FR GB IT SE**

(30) Priorität: **09.06.1994 DE 4420088**

(43) Veröffentlichungstag der Anmeldung:
**26.03.1997 Patentblatt 1997/13**

(73) Patentinhaber: **STOCKHAUSEN GmbH & CO. KG 47805 Krefeld (DE)**

(72) Erfinder:
• **HOUBEN, Jochen**
  **D-47906 Kempen (DE)**
• **HERRMANN, Edgar**
  **D-41334 Nettetal (DE)**
• **DAHMEN, Kurt**
  **D-41239 Mönchengladbach (DE)**

(74) Vertreter: **Klöpsch, Gerald, Dr.-Ing. Patentanwalt Boehmert & Boehmert Anwaltssozietät, Benrather Schlossallee 53 40597 Düsseldorf (DE)**

(56) Entgegenhaltungen:
EP-A- 0 303 518     EP-A- 0 315 185
EP-A- 0 370 646     EP-A- 0 530 438
EP-A- 0 543 303

**Beschreibung**

Die Erfindung betrifft ein Verfahren zur Herstellung von wasserabsorbierenden Flächengebilden, welche aus einem wasserabsorbierenden Polymer und einem vorfabrizierten Nonwoven (Faservlies) bestehen. Das vorfabrizierte Nonwoven wird mit einer Lösung von teilneutralisierter Acrylsäure getränkt, auf eine bestimmte Beschichtungsmenge abgequetscht und anschließend wird die so auf dem Trägermaterial aufgebrachte Monomerlösung polymerisiert, wobei sich das gebildete Polymer und das vorgefertigte Nonwoven innigst verbinden.

Die bisher zur Herstellung dieser In-Situ-Polymerisate beschriebenen Verfahren weisen allesamt den Nachteil auf, daß nach Beendigung der eigentlichen Polymerisation noch ein großer Teil der Monomeren in nichtumgesetzter Form vorliegt. Da diese Monomeren toxikologisch bedenklich sind, werden sie in einem Folgeschritt umgesetzt oder anders entfernt. Dabei bilden sich jedoch im allgemeinen nicht die erwünschten vernetzten Polyacrylate, sondern lineare Polymerketten von mittlerem Molgewicht, die löslich sind und die zur Wasseraufnahme insbesondere unter Druck oder zur WasserRetention unter Druck nicht beitragen können. Ferner haben diese Anteile die anwendungstechnisch unerwünschte Eigenschaft, ein schleimiges Gefühl des In-Situ-Polymerisats nach der Wasseraufnahme zu verursachen. Auch die Verwendung von einem oder mehreren rein thermisch aktivierbaren Startern, vorgeschlagen in EP 257 308, die zusammen mit der Monomerlösung aufgebracht werden, führt nicht zu den gewünschten Produkten, da hierbei aus Sicherheitsgründen eine Mindeststarttemperatur eingehalten werden muß, die deutlich über der einer nicht-thermisch aktivierbaren Radikalbildung liegt. Diese hohe Starttemperatur führt zu kurzen Polymerketten und damit zu anwendungstechnisch schlechten Eigenschaften.

In der EP 123 500, der US 4 443 492 und der EP 054 841 wird ein Waschprozeß zur Nachbehandlung des mittels elektromagnetischer oder korpuskularer ionisierender Strahlung polymerisierten Vlieses beschrieben. Dies führt aber zu einer hohen Abwasserbelastung mit niedermolekularen und unvernetzten Polyacrylsäuren. Die damit erreichte schnellere Wasseraufnahme kann dies nicht ausgleichen.

In der EP 223 908 wird die kontinuierliche Herstellung eines wasserabsorbierenden Flächengebildes beschrieben. Die Polymerisationsreaktion des auf das Vlies aufgebrachten Monomeren wird durch einen großen Überschuß an Radikalbildnern gestartet.Dies führt zu Produkten mit einem sehr hohen Anteil an Restmonomeren und einem geringen Absorptionsverhalten unter Druck. Außerdem ist es schwierig eine Lösung von Acrylsäure und z.B. Kaliumperoxid (Beispiel 1, EP 223 908) aufzubewahren, ohne daß es zu Reaktionen mit der aktivierten Doppelbindung kommt, wodurch der Gehalt der oxidischen Katalysatorkomponente in der Monomerlösung bei der Zwischenlagerung rasch abnimmt.

In der EP 251 314 werden die In-Situ-Polymerisate, die nach der radikalischen Polymerisation Restmonomerengehälter im Prozentbereich aufweisen, mit elektromagnetischer und korpuskularer Strahlung bis zu 100 Mrad bestrahlt. Diese aufwendige und wegen der Sicherheitsmaßnahmen auch teure Technik führt zu einer unkontrollierten Nachvernetzung und zu einer Vergilbung der Produkte. Zwar steigt die Schnelligkeit der Wasseraufnahme, nicht aber gleichzeitig die Wasseraufnahmekapazität.

In der EP 257 308 soll die Senkung der Restmonomeren durch Nacherhitzung des noch feuchten Polymerisates auf 100 - 250°C über einen längeren Zeitraum (15 Minuten) erreicht werden. Dies führt zur Abdestillation der Acrylsäure (Siedepunkt 140°C, Azeotrop mit Wasser bei 99,85°C, Advances in Chemistry, Series 116, Azeotropic Data III, S.16). Dieser Weg, die Restmonomeren zu entfernen, bringt somit erhebliche Probleme bei der Abluftwäsche mit sich und ist auch eine Verschwendung der Ausgangsstoffe. Die im Abluftstrom vorhandene Acrylsäure kann nämlich nicht wiederverwendet werden, da der üblicherweise in der technischen Acrylsäure eingesetzte Polymerisationsinhibitor Hydrochinonmonomethylether sich ebenfalls im Abgas befindet.

In der EP 290 814 wird zur Reduzierung der Restmonomerenmenge die Bestrahlung mit UV-Licht vorgeschlagen, was jedoch einen erhöhten Investitionsbedarf und, bedingt durch das Beschlagen der Lampen, auch einen erhöhten Wartungsaufwand erfordert. Ferner zeigt sich , daß diese Methode zur Restmonomerenvernichtung nur funktioniert, wenn das nachzubehandelnde Polymer einen Wassergehalt von mindestens 20 Gew.-% und außer dem Wasser noch nicht abreagierte Peroxide enthält. Da bei der exothermen Polymerisation der Wassergehalt wegen der großen Oberfläche der In-Situ-Polymerisate stark absinkt, muß also wieder Wasser auf das Vlies aufgebracht werden. Dies ist energetisch und wirtschaftlich ungünstig, da dieses Wasser nach der Bestrahlung wieder abgedampft werden muß.

Aufgabe der Erfindung ist es, die bisher bekannten wasserabsorbierenden Flächengebilde, hergestellt aus einem Faservlies und einem auf dem Faservlies in-Situ erzeugten Polymerisat hinsichtlich der Wasseraufnahme unter Druck, der Retention und des Gehalts an Restmonomeren und löslichen Anteilen zu verbessern und gleichzeitig das Herstellungsverfahren technisch einfach und wirtschaftlich zu gestalten.

Diese Aufgabe wird erfindungsgemäß dadurch gelöst, daß man für die Polymerisation neben den radikalischen, nicht thermisch aktivierbaren Initiatoren, die bevorzugt RedoxSysteme sind, zunächst wenigstens einen thermisch aktivierenbaren Radikalbildner, bevorzugt auf der Basis einer Azoverbindung, einsetzt.

Gegenstand der Erfindung ist somit ein Verfahren zur Herstellung von wasserabsorbierenden Flächengebilden, die aus einem wasserabsorbierenden Polymer und einem vorfabrizierten Faservlies bestehen, wobei das vorfabrizierte Faservlies mit einer Lösung, welche teilneutralisierte Acrylsäure und mindestens einen Vernetzer enthält, getränkt wird, auf eine bestimmte Beschichtungsmenge abgequetscht und anschließend die so auf den Faservlies-Träger aufgebrachte Monomerlösung polymerisiert wird, das dadurch gekennzeichnet ist, daß die Polymerisation in Gegenwart von nicht thermisch aktivierbaren, radikalischen Initiatoren sowie zusätzlich von thermisch zu aktivierenden Radikalbildnern durchgeführt wird.

Bevorzugt wird als nicht thermisch aktivierbarer, radikalischer Starter ein Redox-System verwendet.

Die erfindungsgemäß zusätzlich zum nicht thermisch aktivierbaren Radikalstarter eingesetzten thermisch aktivierbaren Radikalstarter sind beispielsweise Peroxide, die in überschüssiger Menge, bezogen auf die reduzierend wirkende Komponente des Redoxsystems eingesetzt werden, organische Peroxide, insbesondere organische Diperoxide sowie besonders bevorzugt Azoverbindungen mit geeigneter Halbwertszeit.

Der erfindungsgemäß zusätzlich eingesetzte thermisch aktivierbare Radikalstarter kann entweder auf das monomerbeschichtete Vlies aufgesprüht oder wegen der homogenen Verteilung besser direkt als gelöste Verbindung mit der Monomerlösung auf das Trägervlies aufgebracht werden. Der so aufgebrachte thermisch aktivierbare Radikalbildner wird durch die Reaktionswärme der exothermen Polymerisation aktiviert und liefert so die Radikale, die nötig sind, um die Polymerisation fortzusetzen.

Die als thermisch aktivierbare Radikalbildner eingesetzten Verbindungen müssen, um ausreichend wirksam zu sein, so gewählt werden, daß sie in der vorgelegten Monomerlösung nicht, dafür aber im Verlauf der Polymerisation oder nach thermischer Anregung, spätestens jedoch bei 110 °C mit einer Halbwertszeit von weniger als 60 Minuten zerfallen.

Man kann durch die Auswahl des thermisch aktivierbaren Radikal starters den Zeitpunkt einstellen, zu welchem die durch diese Verbindung ausgelöste Polymerisation einsetzt. Dadurch können die anwendungstechnischen Eigenschaften beeinflußt werden. Durch die Menge der homogen im Polymer verteilten thermisch aktivierbaren Starters kann man die Menge der verbleibenden Restmonomeren minimieren. Auch ist es möglich, eine Mischung von mehreren thermisch aktivierbaren Startern in unterschiedlichen Konzentrationen einzusetzen. Aus wirtschaftlichen Gründen sollte nur eine solche Menge an thermisch aktivierbaren Startern zugesetzt werden, wie bei der Monomerenvernichtung noch effektiv wirksam ist.

Als bevorzugte thermisch aktivierbare Starter können Azoverbindungen wie z.B. 2,2'-Azobis[2-(2-imidazolin-2-yl) propane]dihydrochlorid, Azobis (2-Amidinopropan)dihydrochlorid (A.B.A.H.) oder Azo-bis-cyanopentansäure verwendet werden (Vertrieb: Firma Wako). Die Zerfallstemperaturen dieser beispielhaft genannten Azoverbindungen sind bekannt. Obwohl erfahrungsgemäß die Halbwertstemperatur in einer Acrylatlösung um einige Grad höher liegt, kann dieser Wert zur Orientierung dienen.

Als Basismaterial ist jedes offenstrukturierte, flächenförmige Fasergebilde, wie z.B. Vliese und Gewebe geeignet, die nach Druckbelastung aufgrund ihrer Elastizität ein Wiederaufstehvermögen aufweisen. Bevorzugt werden hydrophile Nonwovens, wie z.B. die von der Firma Sandler unter dem Handelsnamen Sawafill vertriebenen Polyesterstapelvliese eingesetzt. Diese Nonwovens werden in Rollen mit einer Breite von 1,50 m oder mehr hergestellt und vertrieben. Als "Nonwoven" werden solche Materialien betrachtet, die auch von der EDANA (European Disposables and Nonwovens Assoziation) in deren Definition einbezogen werden. Da Sauerstoff ein enormes Inhibitionsvermögen für Polymerisationsreaktionen hat und die auf dem Nonwoven aufgebrachte Monomerlösung eine sehr große Oberfläche aufweist, muß durch geeignete technische Maßnahmen der Sauerstoffgehalt vor und während der Polymerisation in der ganzen Apparatur abgesenkt werden. Anzustreben sind Sauerstoffgehalte in der Atmosphäre von unter 100 ppm. Messbar sind solch geringe Sauerstoffspuren z.B. mit Geräten der Firma Systech (Typen EC 91 oder EC 90M) oder Orbisphere (System MOCA).

Als Monomer für das wasserabsorbierende Polymer kommt vor allem Acrylsäure in teilweise neutralisierter Form als Natrium, Kalium oder Ammoniumsalz oder deren Mischung in Frage. Die Zumischung von anderen Monomeren ist möglich. Der Gehalt an Monomeren beträgt üblicherweise 2-8 mol/l. Zur Monomerlösung wird ferner ein Vernetzer in einer Menge von 0,02-1,0 mol% bezogen auf den Monomergehalt, der thermisch aktivierbare Starter oder die Starterkombination in einer Menge von 0,01-0,5 mol% bezogen auf den Monomergehalt sowie die reduzierende Komponente des Redoxpaares (z.B. Ascorbinsäure) , welches die Reaktion einleitet in einer Menge von 0,005-0,1 mol% bezogen auf die Monomeren zugesetzt. Als Vernetzer kommen Verbindungen mit zwei oder mehreren einpolymerisierbaren monoolefinischen oder mit Carboxylgruppen reaktionsfähigen Gruppen oder solche Verbindungen, die mindestens eine mit Carboxylgruppen reaktionsfähige Gruppe und mindestens eine polymerisationsfähige, monoolefinische Gruppe enthalten, wie z.B. Methylenbisacrylamid, Triallylamin, Trimethylolpropantriacrylat, Ethylenglycolbisglycidylether oder der Bismethacrylsäureester von Triethylenglykol um nur einige zu nennen, in Frage. Die Monomerlösung wird bei einer Temperatur von 5-40°C im Foulardbad vorgelegt. Auch sollten etwas Sauerstoff (1-10 ppm) in der Monomerlösung belassen werden, um eine vorzeitige Polymerisation, ausgelöst durch den Zerfall des bevorzugten Azostarters, zu verhindern. Dies ist nötig, da der üblicherweise zugesetzte Stabilisator Hydrochinonmonomethylether nur in Gegenwart von Sauerstoff wirkt.

Das trockene Vlies wird zunächst zur Verdrängung der enthaltenen Luft durch zwei Quetschwalzen durchgeführt und sodann durch einen mit Monomerlösung gefüllten Trog gezogen. Anschließend wird das imprägnierte Nonwoven wiederum durch zwei Quetschwalzen geführt, was den Effekt hat, für eine gleichmäßigere Verteilung der Monomerlösung auf dem Vlies und gleichzeitig für eine konstante und über den Anpreßdruck einstellbare Flottenmenge zu sorgen. Solche Apparate zur Imprägnierung sind aus der der kontinuierlichen Färbereitechnik (Klotzen) bekannt und werden im allgemeinen Foulards genannt. Eine Beschreibung findet man beispielsweise im Ullmann Bd 22, S.711. Außer über den Anpreßdruck kann die aufgebrachte Flottenmenge noch über die Viskosität der Monomerlösung, die Fördergeschwindigkeit, die Hydrophilie und die Dicke des Trägermaterials eingestellt werden. Die Anwendung einer solchen Technik zur Aufbringung von Monomerlösung auf ein vorgefertigtes Substrat ist in der EP 54 841 prinzipiell bereits beschrieben.

Nach Aufbringung der Monomerlösung wird das imprägnierte Gewebe weiterbewegt und durch Aufsprühen der wässrigen Lösung von 0,005-1,0 mol% (bezogen auf Monomer) der Oxidationskomponente die Polymerisation gestartet. Als Oxidationskomponente kommen wasserlösliche oder in Wasser dispegierbare anorganische Oxide wie z.B. Wasserstoffperoxid oder Kaliumpersulfat oder organische Verbindungen wie beispielsweise t-Butylhydroperoxid in Frage. Es wird eine möglichst feine Verteilung dieser Katalysatorkomponente über das imprägnierte Gewebe angestrebt, ohne daß es dabei zum Austreten von unerwünschten Nebeln aus der Apparatur kommt. Über die Düsenform, die Anzahl der Düsen, die Menge des zugesetzten Stickstoffstroms und die Verdünnung mit Wasser läßt sich die Feinheit der Verteilung einstellen. Es kann vorteilhaft sein, die Lösung der Oxidationskomponente in einer vorgekühlten Form (0-20°C) aufzubringen.

Die durch die bevorzugt verwendete Redoxreaktion gestartete exotherme Polymerisation muß mit ausreichendem Umsatz ablaufen, um die bereits mit der Monomerlösung zugesetzten Azoinitiatoren zum Zerfall zu bringen, wodurch es zu einer weiteren Bildung von Radikalen und damit zur weiteren Polymerisation kommt, die die noch verbliebenen Monomeren erfasst. Die maximale Polymerisationstemperatur läßt sich durch die effektive Konzentration der Monomerlösung, die Starttemperatur, die Art und die Konzentration der Redoxstarter einstellen. Auch ist es möglich, den weiteren Fortgang der Polymerisation durch Wärmezufuhr zu unterstützen, womit parallel zur abschließenden Polymerisation der Trocknungsgrad des Endprodukts verbessert werden kann. Geeignet zur Wärmezufuhr ist z.B. überhitzter Wasserdampf, heißer Stickstoff oder Strahlungswärme, wie sie z.B. von IR-Dunkelstrahlern erzeugt wird.

Da die Polymerisation aber auch so geführt werden kann, daß am Ende, z.B. ein trockenes Polymervlies erhalten wird, kann dieses in der erhaltenen Form gelagert, transportiert und weiterverarbeitet werden. Gegebenenfalls wird das zunächst erhaltene erfindungsgemäße Material erneut dem Polymerisationsprozeß zugeführt. Ebenso kann das erfindungsgemäße Material einer oberflächenvernetzenden Nachbehandlung beispielsweise mit Polyolen, Alkylenglykol-glycidylethern und/oder Alkylencarbonaten unterworfen werden. Zur weiteren Verarbeitung können solche Verfahrensschritte, wie z.B. Brechen, Schneiden, Walzen, Stanzen und Aufrollen gehören. Das fertige erfindungsgemäße Material enthält überlicherweise weniger als 20%, vorzugsweise weniger als 10% Wasser. Gegenüber den bisher beschriebenen In-Situ-Polymerisaten zeichnen sich die erfindungsgemäßen Produkte durch eine bessere Wasseraufnahme unter Druck, durch eine bessere Wasserretention unter Druck und durch geringere Gehalte an Restmonomeren und löslichen Anteilen aus.

Das so gebildete In-Situ-Polymerisat findet seinen Einsatz vor allem in der Hygieneindustrie, speziell in Wegwerfwindeln und in Damenbinden sowie im Sanitärbereich. Es ist aber auch eine Verwendung in Pflanzmatten oder bei der Lagerung von flüssigkeitsabsondernden Nahrungsmitteln in flachen Schalen möglich. Die Bahnware kann nach Polymerisation und Trocknung entweder in der Produktionsbreite zu Rollen aufgewickelt oder vorher auf die spätere Verarbeitungsbreite hin geschnitten werden.

<u>Angewendete Testmethoden:</u>

<u>Absorption:</u>

Zur Bestimmung der Absorption werden 5 cm$^2$ des In-Situ-Polymerisates in einen Teebeutel eingeschweißt und für 20 Minuten in 0,9%-iger Kochsalzlösung getaucht. Der Teebeutel wird aus der Lösung genommen und 10 Minuten abtropfen gelassen und gewogen. Einen Teebeutel ohne In-Situ-Polymerisat läßt man als Blindwert mitlaufen:

$$\text{Absorption} = \frac{\text{Auswaage - Blindwert -Einwaage}}{5 \text{ cm}^2}$$

<u>Absorption unter Druck:</u>

Die Aufnahme unter Druck (Druckbelastung 20 g/cm$^2$= 0,3 Psi, bzw 60 g/cm$^2$=0,9 Psi) wird nach einer Modifikation der in der EP 339 461, Seite 7, beschriebenen Methode bestimmt: In einem Zylinder mit Siebboden (Durchmesser 6 cm) wird ein passend ausgeschnittenes kreisförmiges Stück des In-Situ-Polymerisats gelegt und mit einem Stempel belastet, der einen Druck von 20 g/cm² (60 g/cm²) ausübt. Der Zylinder wird anschließend in eine Schale mit 0,9%iger Kochsalzlösung gestellt, wo man den Superabsorber eine Stunde lang 0,9% ige Kochsalzlösung saugen läßt.

$$\text{Absorption unter Druck} = \frac{\text{Auswaage - Einwaage}}{28{,}26\ cm^2}$$

Retention:

Bei der Retention wird wie bei der Absorption verfahren, mit dem Unterschied, daß der Teebeutel nach dem Abhängen in einer Schleuder (23 cm Durchmesser, 1400 Upm) 5 Minuten geschleudert wird.

$$\text{Retention} = \frac{\text{Auswaage - Blindwert - Einwaage}}{5\ cm^2}$$

Lösliche Anteile:

Ca. 1,0 g des In-Situ-Polymerisates werden mit 185 ml 0,9% -iger NaCl-Lösung eine Stunde lang im 250 ml Schlifferlenmeierkolben mittels eines 3 cm langen Rührfisches bei 500 Upm gerührt. Danach wird mittels einer Saugflasche abfiltriert. 20,00 g des Filtrates werden mittels Natronlauge auf den pH 10,0 hochgestellt und mit 0,1n HCl bis auf einen pH von 2,70 titriert. Aus dem Verbrauch der HCl wird der Anteil der löslichen Anteile berechnet, wobei selbstverständlich Comonomereinheiten ohne Carboxylgruppen nicht erfaßt werden und gegebenenfalls zu korrigieren sind..

Restmonomeren:

Die Restmonomeren werden ebenfalls aus obigem Filtrat mittels HPLC-Methode bestimmt und nach dem Verfahren des inneren Standards ausgewertet.

Die Erfindung wird durch die nachfolgenden Beispiele näher erläutert:

Beispiele 1:

0,9282 g (0,0068 mol) Triallylamin, 309,4 g (4,3 mol) Acrylsäure, 311 g Wasser und 240,4 g (3,005 mol) Natronlauge werden unter Kühlung zusammengegeben. Zu dieser Monomerlösung werden die Lösungen von 1,0 g (0,0028 mol) Azobis-(2-Amidinopropan)dihydrochlorid (A.B.A.H.) in 10 g Wasser und 0,45 g (0,0026 mol) Ascorbinsäure in 10 g Wasser gegeben. Diese Monomerlösung mit einem Neutralisationsgrad von 70% und einem Monomergehalt von 4,85 mol/l wird mit Stickstoff solange gespült, bis der restliche Sauerstoffgehalt in der Lösung bei 5-6 ppm liegt. (Messung mit OMI 196, Firma Orbisphere) und bei 20°C im Foulard vorgelegt. Anschließend wird ein 30 cm breites Polyesterstapelvlies (Sawafill 1122, Firma Sandler) in die unter einem leichten Stickstoffüberdruck stehende Anlage eingeführt und durch den Foulard gezogen. Das imprägnierte Vlies wird zwischen zwei Rollen auf die gewünschte Beschichtungsmenge abgequetscht und durch Aufsprühen einer 1%-igen Wasserstoffperoxydlösung die Polymerisation gestartet. Ungefähr eine Minute danach ist die maximale Polymerisationstemperatur erreicht und die Polymerisation wird durch Zuschaltung von IR-Flächenstrahlern (Firma Elstein, Typ HLF, 400 Watt pro Strahler) vervollständigt.

Beispiel 2:

Es wurde genauso vorgegangen wie oben, nur wurde statt der oben angegebenen Menge an Azobis-(2-Amidinopropan)dihydrochlorid eine Kombination von 0,71 g (0,002 mol) Azobis-(2-Amidinopropan)dihydrochlorid und 0,56 g (0,002 mol) Azocyanopentansäure eingesetzt.

Beispiel 3:

Es wurde wie im Beispiel 2 vorgegangen, nur wurde die Azocyanopentansäure durch 0,65 g 2,2'-Azobis[2-(2-imidazolin-2-yl)propanedihydrochlorid ersetzt und die Starter erst bei einer Temperatur von 5°C zur Monomerlösung gegeben und diese auch bei dieser Temperatur aufbewahrt. Die Reaktion wurde durch Aufsprühen einer 2%-igen Wasserstoffperoxidlösung ausgelöst. Im Anschluß an die Polymerisation wurde das In-Situ-Polymerisat mit einem vorgewärmten Stickstoffstrom getrocknet.

Beispiel 4:

135,7 g Acrylsäure (1,88 mol), 164,3 g KOH (45%-ig) (1,32 mol), 0,6 g Triallylamin, 0,3 g Trimethylolpropantriacrylat und 0,5 g Ascorbinsäure werden unter Kühlung zusammengegeben. Dazu wird eine Lösung von 0,45 g A.B.A.H. in 2 g Wasser gegeben. Diese Lösung mit einer Monomerkonzentration von 6,2 mol/l und einem Neutralisationsgrad von 70% wurde bei 30°C aufgebracht. Durch Aufsprühen einer 1%-igen Lösung von t-Butylhydroperoxyd wurde die Reaktion ausgelöst und ohne weitere Energiezufuhr zu Ende geführt.

Beispiel 5:

Es wurde wie im Beispiel 1 vorgegangen, nur wurde statt des Triallylamins 2,4 g (0,01 mol) Diethylenglycoldimethacrylat (Bisomer DEGMA, Hersteller ISC) eingesetzt.

Beispiel 6:

Es wurde wie im Beispiel 2 vorgegangen, nur wurde die Mengen der beiden Azoverbindungen verdreifacht.

Vergleichsbeispiele:

Beispiel 7:

Es wurde wie im Beispiel 1 vorgegangen mit der Ausnahme, daß die Azoverbindung A.B.A.H. ersatzlos weggelassen wurde.

Beispiel 8 (entspricht Beispiel 1 aus EP 257 308)

250 g (3,125 mol) 50%-ige Natronlauge, 265 g Wasser, 300 g (4,17 mol) Acrylsäure und 0,3 g (0,002 mol) N,N'-Methylenbisacrylamid wurden unter Eiskühlung zusammengegeben. Die wässrige Lösung hatte einen Neutralisationsgrad von 75% und eine Monomerkonzentration von 5,1 mol/l. Die Lösung wurde mit Stickstoff inertisiert und auf 50°C aufgeheizt Sodann wurden 0,6 g (0,002 mol) Kaliumpersulfat zugesetzt. Diese Lösung wurde bei 50°C im Foulard vorgelegt und ein Polyesterstapelvlies damit imprägniert. Durch Aufsprühen einer 5%-igen Lösung von Natriumhydrogensulfit in Wasser wurde die Polymerisation gestartet, welche aber erst nach Aufbringung von 16 g der Hydrogensulfitlösung ansprang. Der Feuchtigkeitsgehalt wurde auf 20% eingestellt und das In-Situ-Polymerisat wurde 15 Minuten bei 170°C in einen Umlufttrockenschrank erhitzt. Man erhält ein gelbliches, hartes Vlies.

Beispiel 9 (entspricht Beispiel 2 aus EP 223 908)

324 g (4,5 mol) Acrylsäure, 222 g (3,36 mol) feste 85%-ige KOH, 180 g Wasser und 1,0 g (0,006 mol)N,N'-Methylenbisacrylamid wurden unter Kühlung zusammengegeben. Zum Schluß wurden noch 1,9 g 35%-iges (0,0175 mol) Wasserstoffperoxid zugesetzt. Mit dieser Lösung wurde das oben beschriebene Polyestervlies beschichtet. Das imprägnierte Vlies wurde mittels IR-Dunkelstrahlern (Elstein) auf 100°C vorgeheizt und mit einer 7%-igen Lösung von Monoethanolamin besprüht, woraufhin die Polymerisation einsetzte.

Die erhaltenen Produkte wurden auf ihre Eigenschaften untersucht; die Ergebnisse sind in Tabelle 1 dargestellt.

ITabelle 1: Prüfergebnisse der Beispiele 1-9:

| Beispiele | 1 | 2 | 3 | 4 | 5 | 6 | 7 | 8 | 9 |
|---|---|---|---|---|---|---|---|---|---|
| Polymerbeladung | 195 g/m2 | 190 g/m2 | 200 g/m2 | 195 g/m2 | 200 g/m2 | 195 g/m2 | 195 g/m2 | 185 g/m2 | 200 g/m2 |
| Absorption | 4450 g/m2 | 4560 g/m2 | 4870 g/m2 | 3860 g/m2 | 7780 g/m2 | 4830 g/m2 | 6100 g/m2 | 6200 g/m2 | 5950 g/m2 |
| Retention | 3705 g/m2 | 3800 g/m2 | 4050 g/m2 | 3220 g/m2 | 4980 g/m2 | 3750 g/m2 | 1320 g/m2 | 1450 g/m2 | 2840 g/m2 |
| AUL 0,3 Psi | 3100 g/m2 | 3200 g/m2 | 3200 g/m2 | 3110 g/m2 | 4740 g/m2 | 3160 g/m2 | 1040 g/m2 | 1130 g/m2 | 1250 g/m2 |
| AUL 0,9 Psi | 1250g/m2 | 1340 g/m2 | 1320 g/m2 | 1650 g/m2 | 1130 g/m2 | 1380 g/m2 | <500 g/m2 | <500 ppm | <500 ppm |
| lösliche Anteile | 3,10% | 4,00% | 4,10% | 3,90% | 3,70% | 1,80% | 18% | 9,50% | 5,50% |
| Restmonomere | 450 ppm | 280 ppm | 320 ppm | 420 ppm | 430 ppm | 180 ppm | 6500 ppm | 400 ppm | 5800 ppm |
| Feuchte | 4,50% | 4,50% | 4,40% | 4,70% | 4,35% | 4,40% | 6,30% | 2,10% | 4,30% |

# EP 0 764 223 B1

## Patentansprüche

1. Verfahren zur Herstellung von wasserabsorbierenden Flächengebilden, die aus einem wasserabsorbierenden Polymer und einem vorfabrizierten Faservlies bestehen, wobei das vorfabrizierte Faservlies mit einer Lösung, welche teilneutralisierte Acrylsäure und mindestens einen Vernetzer enthält, getränkt wird, auf eine bestimmte Beschichtungsmenge abgequetscht und anschließend die so auf den Faservlies-Träger aufgebrachte Monomerlösung polymerisiert wird, dadurch gekennzeichnet, daß die Polymerisation der Monomerlösung bei einem Restgehalt von 1 bis 10 ppm Sauerstoff in der Monomerlösung in Gegenwart eines Redoxsystems und eines thermisch aktivierbaren Radikalbildners durchgeführt wird, wobei die reduzierende Komponente in der Monomerlösung enthalten ist, und die oxidierende Komponente auf das mit Monomerenlösung getränkte Faservlies aufgesprüht wird.

2. Verfahren nach Anspruch 1, dadurch gekennzeichnet, daß als nicht thermisch aktivierbare Radikalstarter ein Redoxsystem verwendet wird.

3. Verfahren nach Anspruch 1 oder 2, dadurch gekennzeichnet, daß als thermisch aktivierbarer Radikalbildner wenigstens eine Azoverbindung verwendet wird.

4. Verfahren nach einem der Ansprüche 1 - 3, dadurch gekennzeichnet, daß der thermisch aktivierbare Radikalstarter eine Halbwertszeit von kleiner oder gleich 60 Minuten bei 110 °C aufweist.

5. Verfahren nach Anspruch 3, dadurch gekennzeichnet, daß als Azoverbindungen 2,2'-Azobis[2-(2-imidazolin-2-yl) propane]dihydrochlorid, Azobis (2-Amidinopropan)dihydrochlorid (A.B.A.H.) und/oder Azo-bis-cyanopentansäure verwendet werden.

6. Verfahren nach einem der Ansprüche 1 - 5, dadurch gekennzeichnet, daß der thermisch aktivierbare Radikalbildner in Mengen von 0,01 bis 0,5, bevorzugt 0,01 bis 0,1 Mol%, bezogen auf die Monomerenmenge, eingesetzt wird.

7. Verfahren nach einem der Ansprüche 1 - 6, dadurch gekennzeichnet, daß als polymerisierbare Monomere Acrylsäure, Methacrylsäure und/oder die Natrium-, Kalium- und Ammoniumsalze dieser Säuren verwendet werden.

8. Verfahren nach einem der Ansprüche 1 - 7, dadurch gekennzeichnet, daß die Polymerisation in Gegenwart wenigstens eines, mindestens zwei funktionelle monoolefinische oder mit Carboxylgruppen reaktionsfähige Gruppen enthaltenden Vernetzers durchgeführt wird.

9. Verwendung der Flächengebilde nach einem der Ansprüche 1 - 7 in Hygieneprodukten, insbesondere in Wegwerfwindeln und in Damenbinden sowie allein oder als Komponente im Sanitärbereich.

10. Verwendung der Flächengebilde nach einem der Ansprüche 1 - 8 in Pflanzmatten und in Lagerschalen, die der Lagerung von flüssigkeitsabsondernden Nahrungsmitteln dienen.

## Claims

1. A process for the production of water-absorbing sheet materials consisting of a water-absorbing polymer and a prefabricated nonwoven fabric, wherein said prefabricated nonwoven fabric is soaked with a solution containing partially neutralized acrylic acid and at least one crosslinker, squeezed off to a specific coating amount, followed by polymerizing the monomer solution thus coated onto said nonwoven fabric support, characterized in that the polymerization of the monomer solution is performed at a residual oxygen content in said monomer solution of from 1 to 10 ppm in the presence of a redox system and a thermally activatable free radical former, the reducing component being included in the monomer solution, and the oxidizing component being sprayed onto said nonwoven fabric soaked with monomer solution.

2. The process according to claim 1, characterized in that a redox system is used as thermally non-activatable free radical former.

3. The process according to claim 1 or 2, characterized in that at least one azo compound is used as thermally activatable free radical former.

4. The process according to one of claims of 1 through 3, characterized in that the thermally activatable free radical initiator has a half-life of below or equal to 60 minutes at 110°C.

5. The process according to claim 3, characterized in that 2,2'-azobis[2-(2-imidazolin-2-yl)propane] dihydrochloride, azobis(2-amidinopropane) dihydrochloride (A.B.A.H.), and/or azobis(cyanopentanoic acid) are used as azo compounds.

6. The process according to one of claims 1 through 5, characterized in that the thermally activatable free radical former is used in amounts of from 0.01 to 0.5, preferably from 0.01 to 0.1 mole-%, relative to the amount of monomers.

7. The process according to one of claims 1 through 6, characterized in that acrylic acid, methacrylic acid and/or the sodium, potassium and ammonium salts of these acids are used as polymerizable monomers.

8. The process according to one of claims 1 through 7, characterized in that the polymerization is carried out in the presence of at least one crosslinker containing at least two functional groups which are monoolefinic or reactive with carboxyl groups.

9. Use of the sheet materials according to one of claims 1 through 7 in hygienic products, particularly in disposable diapers and in liners, and alone or as a component for sanitary use.

10. Use of the sheet materials according to one of claims 1 through 8 in planting pads and in storing trays for foodstuffs exuding liquid.

**Revendications**

1. Procédé de fabrication d'articles plats absorbant l'eau, qui se composent d'un polymère absorbant l'eau et d'une nappe de fibres préfabriquées, où l'on imprègne la nappe de fibres préfabriquées d'une solution qui contient de l'acide acrylique partiellement neutralisé et au moins un agent de réticulation, on l'exprime jusqu'à une quantité de revêtement déterminée et on polymérise ensuite la solution du monomère appliquée sur le support constitué de la nappe de fibres, caractérisé en ce que l'on entreprend la polymérisation de la solution du monomère à une teneur résiduelle de 1 à 10 ppm d'oxygène dans la solution du monomère, en présence d'un système redox et d'un agent formateur de radicaux thermo-activable, où le composant réducteur est contenu dans la solution du monomère et le composant oxydant est pulvérisé sur la nappe de fibres imprégnée de la solution du monomère.

2. Procédé suivant la revendication 1, caractérisé en ce que l'on utilise un système redox, à titre d'amorceur radicalaire thermo-activable.

3. Procédé suivant la revendication 1 ou 2, caractérisé en ce que l'on utilise au moins un composé du type azo, à titre d'agent formateur de radicaux thermo-activable.

4. Procédé suivant l'une quelconque des revendications 1 à 3, caractérisé en ce que l'amorceur de radicaux thermo-activable présente une demi-durée de vie, inférieure ou égale à 60 minutes à 110°C.

5. Procédé suivant la revendication 3, caractérisé en ce que l'on utilise, à titre de composés du type azo, le dichlorhydrate de 2,2'-azobis[2-(2-imidazoline-2-yl)propane], le dichlorhydrate d'azobis(2-amidinopropane) (A.B.A.H.) et/ou l'acide azo-bis-cyanopentanoïque.

6. Procédé suivant l'une quelconque des revendications 1 à 5, caractérisé en ce que l'on utilise l'agent formateur de radicaux thermo-activable en proportions de 0,01 à 0,5% molaire, de préférence, 0,01 à 0,1% molaire, par rapport à la quantité du monomère.

7. Procédé suivant l'une quelconque des revendications 1 à 6, caractérisé en ce que l'on utilise, à titre de monomère polymérisable, de l'acide acrylique, de l'acide méthacrylique et/ou les sels de sodium, de potassium et d'ammonium de ces acides.

8. Procédé suivant l'une quelconque des revendications 1 à 7, caractérisé en ce que l'on entreprend la polymérisation en présence d'un agent de réticulation contenant au moins deux radicaux monooléfiniques fonctionnels ou susceptibles de réagir avec des radicaux carboxyle.

9. Utilisation des articles plats suivant l'une quelconque des revendications 1 à 7 dans des produits destinés à l'hygiène, en particulier, dans des couches à jeter et dans des serviettes hygiéniques, comme aussi seuls ou à titre de composants dans le domaine sanitaire.

10. Utilisation des articles plats suivant l'une quelconque des revendications 1 à 8 dans des paillis pour plantes et dans des plateaux de conservation qui servent à la conservation de produits alimentaires absorbant des liquides.